# EUROPEAN PATENT APPLICATION

(11) **EP 4 696 313 A1**
(43) Date of publication of application: **18.02.2026**
(21) Application number: 24194432.1
(22) Date of filing: 13.08.2024
(51) Int. Cl.: A61K 35/612, A61K 36/28, A61K 31/357, A61P 1/16

(54) **FORMULATION OF KRILL OIL AND SILYMARIN**

(71) Applicant: WÖRWAG Pharma GmbH & Co.KG, 71034 Böblingen (DE)
(72) Inventor: Reule, Claudia, 71686 Remseck a. N. (DE); Guerrero, Gregorio, 70199 Stuttgart (DE); Wollmann, Jan-Christoph, 55545 Bad Kreuznach (DE)
(74) Representative: Simandi, Claus

(57) **Abstract**

The invention relates to a novel formulation or composition of krill oil and silymarin and use thereof, in particular for the treatment and prophylaxis of liver diseases.

## Description

The invention relates to a novel formulation or composition of krill oil and silymarin and use thereof, in particular for the treatment and prophylaxis of liver or bile diseases.

The milk thistle (Silybum marianum or Carduus marianus) is a cultivated plant that is particularly widespread in south-western and central Europe and has naturalised in Eurasia, North America and South America.

The drug is known to be effective in the prevention and treatment of various forms of liver and gallbladder dysfunction, as well as cancer and tumour diseases. The drug consists of the ripe fruits, freed from the pappus, with a minimum silymarin content of 1.5%. Tinctures (usually alcoholic-aqueous extracts of the drug) from milk thistle have been known since ancient times. In particular, isolated silymarin is particularly important and suitable (e.g. DE 1 923 082 (Madaus)). Milk thistle extracts are common medicines and are also an ingredient of combination preparations, such as the phytopharmaceutical Iberogast^{®}. A well-known milk thistle fruit dry extract product is Legalon forte^{®}, for example.

Silymarin is a flavonolignan complex or polyhydroxyphenylchromanone and was first isolated from the plant in the 1960s (dissertation by Janiak Bernhard, June 1960, FU-Berlin (DE 2020407), Pelter A., Hänsel R., Tetrahedron Letters, 25, (1968)).

Silymarin consists of an isobaric mixture of flavonolignans with the main components silybin A and B (silibinin A and B), isosilybin A and B (isosilibinin A and B), silydianin (silidianin) and silychristin (silichristin) (cf. Kim, N.-C.; Graf, T. N.; Sparacino, C. M.; Wani, M. C.; Wall, M. E., Complete isolation and characterization of silybins and isosilybins from milk thistle (Silybum marianum). Organic & Biomolecular Chemistry 2003, 1, 1684-1689.; Smith, W. A.; Lauren, D. R.; Burgess, E. J.; Nigel, B. P.; Martin, R. J., A Silychristin Isomer and Variation of Falavonolignan Levels in Milk Thistle (Silybum marianum) Fruits Planta Medica 2005, 71, 877-880).

Other known secondary constituents are 2,3-dehydrosilybin, desoxysilydianin (silymonin), silandrin, silybinom, silyhermin and neosilyhermin.

The declared requirements for a dry milk thistle extract are a content of preferably 65-85% by weight of silymarin (other ranges are possible), with the silymarin content being composed as follows:
40-65% by weight (relative proportion of total silymarin):
   silybin A and B (synonymously silibinin) (diastereomeric mixture, C₂₅H₂₂OH₁₀ MW 482.4 (CAS 22888-70-6)) and
10-20 wt.% (relative proportion of total silymarin):
   isosilybin A and B (diastereomeric mixture, C₂₅H₂₂OH₁₀ MW 482.4 (CAS 72581-71-6)) and
20-45% by weight (relative proportion of total silymarin):
   silydanin and silychristin (C₂₅H₂₂OH₁₀ MW 482.4) .

These flavonolignans are insoluble or difficult to dissolve in water (the solubility of pure silymarin at pH 6.9 is approx. 0.08 mg/ml) and have low bioavailability.

In the context of the present invention, "silymarin" is a mixture of substances which contains (at least) the four substances silybin, silydianin, silychristin and isosilybin in different concentrations. It is immaterial in which proportions these substances are present in relation to each other and whether other substances are present in the mixture. This variation may depend on the origin and the genotype and phenotype of the milk thistle used (plant drug). However, it is preferable that these substances fulfil the requirements of the Ph. Eur. or DAB in the respective applicable version. According to the invention, this is the case.

Krill oil is a dietary supplement derived from tiny shrimp-like crustaceans called krill, which are found in the oceans, particularly in the Antarctic. Krill oil is made through a series of processes that involve harvesting, extraction, and purification. The krill are then subjected to extraction processes to obtain the oil. Common methods include solvent extraction and cold-pressing. Solvent extraction involves using food-grade solvents to separate the oil from the krill, while cold-pressing physically extracts the oil without the use of heat or chemicals.

The krill belong to the order Euphausiacea, and one of the most well-known species is the Antarctic krill (Euphausia superba). These tiny creatures play a crucial role in the marine ecosystem as they are a major food source for many larger animals.

Krill are typically about 1 to 2 centimeters long and have a semi-transparent body. They feed primarily on phytoplankton, microscopic plants that drift near the ocean surface. Through their feeding activities, krill play a key role in the ocean's carbon cycle, helping to transport carbon from the surface waters to the deep sea.

Krill oil is rich in omega-3 fatty acids, primarily eicosapentaenoic acid (EPA) and docosahexaenoic acid (DHA), which are also found in fish oil. These omega-3 fatty acids are known for their potential health benefits, including supporting heart health, reducing inflammation, and improving brain function.

One of the key differences between krill oil and fish oil is that the omega-3 fatty acids in krill oil are attached to phospholipids, which may make them more easily absorbed by the body compared to the triglyceride form found in fish oil. Additionally, krill oil contains astaxanthin, a powerful antioxidant that can help protect the oil from oxidation and add extra health benefits.

Because krill are lower on the food chain, they accumulate fewer toxins than larger fish, which can be an advantage over some fish oils.

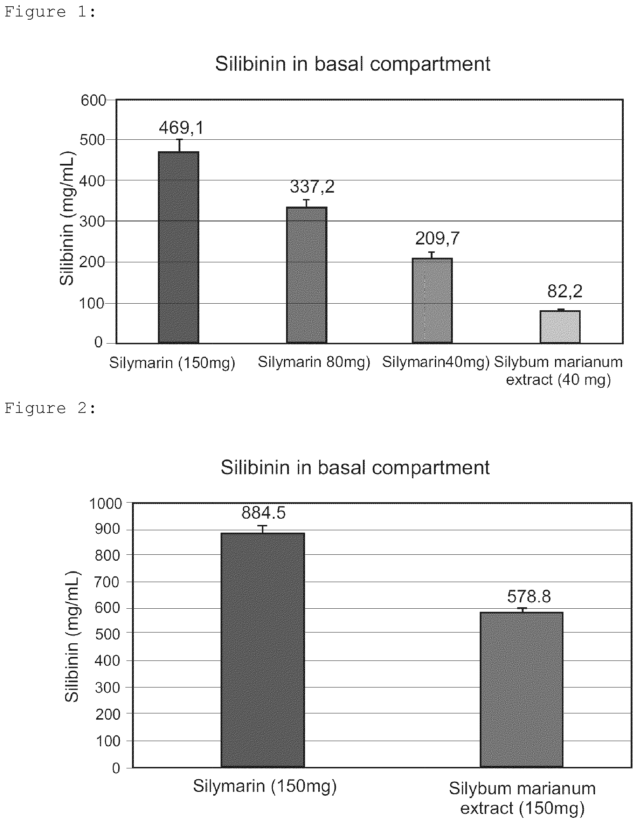

Now, the inventors have found that krill oil improves or enhances the bioavailability of silymarin respectively silybin, namely about 255 % (see Example 2, Figure 1). It is stated by the inventors that, surprisingly, choline - the active ingredient in krill oil - plays an important role in the accumulation of silybin in the blood stream.

A preferred krill oil in accordance with the invention can be characterized by the following features:

| | |
|---|---|
| Total phospholipids, incl. phosphatidylcholine | >= 56 g/100 g |
| Total omega 3 fatty acids | >= 27 g/100 g |
| Choline | >= 7 g/100 g |

In a further preferred embodiment krill oil is solely derived from Euphausia superba.

In a further preferred embodiment according to the invention, an additional amount of, in particular, 100 mg to 2,000 mg of choline is added to, in particular, 100 g of the formulation or composition, preferably in the form of a salt, for example choline bitartrate. The choline salt is completely dissolved.

The problem addressed by the present invention is therefore to provide a novel formulation or composition for improving or enhancing the bioavailability of silybin in a subject.

The object or problem is solved by the technical teaching as provided in at least one of the claims.

For this purpose, the applicant has performed comprehensive tests with several weight ratio of silymarin to krill oil (see examples and drawings).

Surprisingly, for a composition of silymarin and krill oil the bioavailability of silymarin can be improved or enhanced in a subject as outlined (supra).

Hence, the present invention provides a composition comprising:
(a) milk thistle extract containing silymarin, and
(b) krill oil,
wherein a weight ratio (w/w) of silymarin to the krill oil is 1 : 2 to 1 : 40, in particular 1 : 5 to 1 : 30.

In a further preferred embodiment of the invention the composition comprises:
(a) milk thistle extract containing silymarin, and
(b) krill oil,
wherein a weight ratio (w/w) of silymarin to the krill oil is 1 : 8 to 1 : 20, in particular 1 : 10 to 1 : 15.

Other preferred weight ratios (w/w) of silymarin to the krill oil is: 1 : 8 to 1 : 10, 1 : 10 to 1 : 20, 1 : 13 to 1 : 17, 1 : 20 to 1 : 35 or 1 : 25 to 1 : 30.

In a further preferred embodiment of the invention the composition comprises:
(a) milk thistle extract containing silymarin, and
(b) krill oil,
wherein a weight ratio (w/w) of silymarin to the krill oil is 35 mg - 160 mg : 450 mg - 1200 mg, in particular 35 mg - 160 mg : 450 mg - 700 mg, in particular 51 mg : 570 mg, in particular 40 mg - 80 mg : 1000 mg - 1350 mg, or 80 mg - 150 mg : 1000 mg - 1350 mg, in particular 40 mg - 80 mg : 400 mg - 800 mg or 80 mg - 150 mg : 400 mg - 800 mg.

Other preferred weight ratios (w/w) of silymarin to the krill oil are: 40 mg : 1200 mg (1 : 30), 80 mg : 1200 mg (1 : 15), 150 mg : 1200 mg (1 : 8).

In a further preferred embodiment of the invention the milk thistle extract contains 65-85% by weight of silymarin.

In the context of this invention, liver diseases and disorders are preferably selected from the group consisting of hepatitis, Steatotic Liver Disease (SLD), Metabolic Dysfunction-associated Steatotic Liver Disease (MASLD), Metabolic Dysfunction-associated Steatohepatitis (MASH) (formerly: Non-Alcoholic Fatty Liver Disease (NAFLD), Non-Alcoholic Fatty Liver (NAFL), Non-Alcoholic Steatohepatitis (NASH)), Alcoholic Liver Disease (ALD) or cirrhosis.

In the context of this invention, bile diseases and disorders refer to any inflammation of the gall, gallbladder or bile duct.

All of said indications are described in Pschyrembel, 268th edition 2023, De Gruyter (Berlin) and further information are available by the European Association for the Study of the Liver (EASL).

The effective agent containing the novel formulation or composition according to the invention can now advantageously be used for the treatment and prophylaxis of an ill patient or individual, subject or preferably human, and specifically for the treatment and prophylaxis of liver diseases and disorders, preferably selected from the group consisting of hepatitis, Steatotic Liver Disease (SLD), Metabolic Dysfunction-associated Steatotic Liver Disease (MASLD), Metabolic Dysfunction-associated Steatohepatitis (MASH) (formerly: Non-Alcoholic Fatty Liver Disease (NAFLD), Non-Alcoholic Fatty Liver (NAFL), Non-Alcoholic Steatohepatitis (NASH)), Alcoholic Liver Disease (ALD) or cirrhosis.

The agents can be administered in any desired quantities and dosages.

The galenic formulation of the agent according to the invention can be selected from the group consisting of in the form of drops, capsules, soft capsules or gel capsules or infusions, preferably for oral applications. The formulation may of course contain pharmaceutically standard excipients.

In another embodiment, the invention relates to a drug containing an agent according to the invention for use or application for the treatment and prophylaxis of liver diseases and disorders, preferably selected from the group consisting of hepatitis, Steatotic Liver Disease (SLD), Metabolic Dysfunction-associated Steatotic Liver Disease (MASLD), Metabolic Dysfunction-associated Steatohepatitis (MASH)(formerly: Non-Alcoholic Fatty Liver Disease (NAFLD), Non-Alcoholic Fatty Liver (NAFL), Non-Alcoholic Steatohepatitis (NASH)), Alcoholic Liver Disease (ALD) or cirrhosis..

Another preferred embodiment relates to a dietary supplement containing the agent according to the invention, in particular in the form of a dietary composition or balanced diet for the treatment and prophylaxis of liver diseases and disorders, preferably selected from the group consisting of hepatitis, Steatotic Liver Disease (SLD), Metabolic Dysfunction-associated Steatotic Liver Disease (MASLD), Metabolic Dysfunction-associated Steatohepatitis (MASH) (formerly: Non-Alcoholic Fatty Liver Disease (NAFLD), Non-Alcoholic Fatty Liver (NAFL), Non-Alcoholic Steatohepatitis (NASH)), Alcoholic Liver Disease (ALD) or cirrhosis. A suitable physiologically compatible carrier can be added to the dietary supplement according to the invention.

The pharmaceutical preparations according to the invention may be prepared in the form of dosage units or formulation units. This means that the preparations are in the form of individual parts, preferably capsules and vials, the active-substance content of which corresponds to a multiple of an individual dose. The dosage units may e.g. contain 1, 2, 3 or 4 individual doses or 1/2, 1/3 or 1/4 of an individual dose. An individual dose preferably contains the quantity of the agent according to the invention that is administered in an application and usually corresponds to a whole daily dose, half of a daily dose, a third of a daily dose or a quarter of a daily dose. Preferably, a dose is administered three times per day, preferably in the form of a capsule or drops, in particular in the morning, at lunchtime and in the evening, optionally at mealtimes.

Therefore, the invention likewise relates to a pharmaceutical preparation containing an agent according to the invention together with excipients and additives.

It should be noted that features described in connection with an exemplary embodiment or an exemplary article may be used with any other exemplary embodiment or with any other exemplary article can be combined.

If a term is designated with an indefinite or definite article, such as "a" in the singular, this also includes the term in the plural and vice versa, unless the context clearly states otherwise.

The term "comprise" as used herein not only includes the meaning of "contain" but can also mean "consist of" and "consisting essentially of".

### Examples and drawings:

The following examples and drawings (figures) are used solely to explain the invention, without limiting the invention to these examples.

### Example 1:

The following test products were used:

**Table 1: Test substances and batch numbers**

| Product | Batch | Batch number |
|---|---|---|
| Formule 1 | Silybum marianum L. (80% Silymarin) (150 mg) + Krill oil | 2033-1 |
| | Superba boost (1,2 g) | |
| | Manufacturing date: 11/12/2023 | |
| Formule 2 | Silybum marianum L. (80% Silymarin) (80 mg) | 2033-2 |
| | Krill oil Superba boost (1,2 g) | |
| | Manufacturing date: 11/12/2023 | |
| Formule 3 | Silybum marianum L. (80% Silymarin) (40 mg)+ Krill oil | 2033-3 |
| | Superba boost (1,2 g) | |
| | Manufacturing date: 11/12/2023 | |
| Reference | Silybum marianum L. (80% Silymarin) | 2033-4 |
| | Extract Batch C20221008; | |
| | Manufacturing date: 08/10/2022; | |
| | Gonmisol Fine Ingredients | |

### In vitro digest simulation

The in vitro model "artificial digest" simulated the enzymatic and pH conditions during the gastro-intestinal-passage. Metabolic changes of the active ingredients in the test product due to these conditions will be considered in the subsequent examples.

Therefore, the respective test product was added to an aqueous mucin/pepsin solution at a pH of 2 for 2 h at 37 °C. After adding pankreatin, trypsin, and bile extracts the pH of the mixture was adjusted to pH 7,5. The respective solutions were incubated for further 4 h at 37 °C.

Digest solutions of formule 1-3 and Silybum marianum extract can be used in a concentration of 2%.

### Example 2:

The in vitro assessment of the intestinal transepithelial transport of silymarin was performed using the well-established Caco-2 model. The Caco-2 cell line is derived from human epithelial colorectal adenocarcinoma cells and is commonly used as model for intestinal uptake. The cells are seeded on filter inserts and differentiated over 14-21 days for full performance.

In a first step, a pre-test was made to check the sensitivity of the analytical method to make sure that the silymarin concentration tolerated by the CaCo-2 model as well as in the basolateral compartment is still above the detection limit.

### Main Assay

The transepithelial transport of silybin from formule 1, 2 and 3 as well as from Silybum marianum extract was determined for the respective 2% digestion solution.

After 24 h incubation the silybin content in the basal compartment was determined by LC-MS/MS.

Silybin was not detectable in the digestion control samples. As can be seen in Figure 1, the concentration of silybin in the basal compartment is dependent on the concentration applied on top.

If formule 3 is compared with the corresponding initial concentration Silybum marianum extract (40 mg) the basal concentration is increased by approximately 2.5-fold (255%).

Based on the data obtained, the following apparent permeability coefficient (Pₐₚₚ) values for the permeation of silybin from the apical to basolateral compartment were calculated (Artursson P., Karlsson J., Biochem Biophys Res Commun., 175(3), 880-885 (1991)).

**Table 2: Pₐₚₚ values of the formulations.**

| | Pₐₚₚ Silybin [x 10E-7 cm/s] |
|---|---|
| Silymarin (150mg) in krill oil / formule 1 | 5.02 ± 0.34 |
| Silymarin (80mg) in krill oil / formule 2 | 4.54 ± 0. 19 |
| Silymarin (40mg) in krill oil / formule 3 | 3.80 ± 0.22 |
| Silybum marianum extract (40mg) | 2.96 ± 0.12 |

There is a dose-dependent increase in transport rate with increasing silymarin concentration.

Comparing Pₐₚₚ for formule 3 with that for the corresponding initial concentration of Silybum marianum extract concentration, there is an increase by approximately 28% in the transport rate of the formulation in krill oil.

For the other Silymarin components, silicristin, and isosilibin, no quantitative determination was made. However, different transport rates were observed for the other components, leading to a shift in their ratio to each other.

The ratio of silicristin : silibinin (silybin) : isosilibin in the supplemented solution was 19.2 - 26.8 : 46.8 - 55.3 : 21.6 - 26.4,
in the basal compartment: 3.7 - 5.0 : 75.7 - 81.3 : 14.8 - 19.2

From these observations it can be concluded that silicristin is rarely transported across the epithelial barrier.

### Example 3:

A comparison of formule 1 (150 mg in krill oil) with corresponding initial concentration of Silybum marianum extract (150 mg) was made. As can be seen in Figure 2, the silybin concentration in the basal compartment is higher in the group with the krill oil formulation than in Silybum marianum extract group.

If formule 1 is compared with the corresponding initial concentration of Silybum marianum extract (150 mg) the basal concentration is increased by approximately 1.5-fold.

Based on the data obtained, the following apparent permeability coefficient (Pₐₚₚ) values for the permeation of silybin from the apical to basolateral compartment were calculated.

**Table 3: Pₐₚₚ values of the different formulations.**

| | Pₐₚₚ Silybin [x 10E-7 cm/s] |
|---|---|
| Silymarin (150 mg) in krill oil / formule 1 | 5.63 ± 0.99 |
| Silybum marianum extract (150mg) | 5.06 ± 0.32 |

Comparing Pₐₚₚ for formule 1 with that for the corresponding initial concentration of Silybum marianum extract concentration, there is an increase by approximately 11% in the transport rate of the formulation in krill oil.

Figure 1: Transepithelial transport of silybin (resp. silibinin) using Caco-2 models. Silybin concentration in the basal compartment after 24 h incubation with the respective digestion solution (2%).

Values are expressed in ng/ml, mean ± SD, n=6.

Figure 2: Transepithelial transport of silybin (resp. silibinin) using Caco-2 models. Silybin concentration in basal compartment after 24 h incubation with the respective digestion solution {2%).

Values are expressed in ng/ml, mean ± SD, n=6.

## Claims

1. A composition comprising:
(a) milk thistle extract containing silymarin, and
(b) krill oil,
wherein a weight ratio (w/w) of silymarin to the krill oil is 1 : 2 to 1 : 40, in particular 1 : 5 to 1 : 30.

2. The composition of claim 1 comprising:
(a) milk thistle extract containing silymarin, and
(b) krill oil,
wherein a weight ratio (w/w) of silymarin to the krill oil is 1 : 8 to 1 : 20, in particular 1 : 10 to 1 : 15.

3. The composition of claim 1 comprising:
(a) milk thistle extract containing silymarin, and
(b) krill oil,
wherein a weight ratio (w/w) of silymarin to the krill oil is 1 : 8 to 1 : 10, 1 : 10 to 1 : 20, 1 : 13 to 1 : 17, 1 : 20 to 1 : 35 or 1 : 25 to 1 : 30.

4. The composition of claims 1 to 3 comprising:
(a) milk thistle extract containing silymarin, and
(b) krill oil,
wherein a weight ratio (w/w) of silymarin to the krill oil is 35 mg - 160 mg : 450 mg - 1200 mg, in particular 35 mg - 160 mg : 450 mg - 700 mg, in particular 51 mg : 570 mg, in particular 40 mg - 80 mg : 1000 mg - 1350 mg, or
80 mg - 150 mg : 1000 mg - 1350 mg, in particular 40 mg - 80 mg : 400 mg - 800 mg or 80 mg - 150 mg : 400 mg - 800 mg.

5. A composition according to any of claims 1 to 4 wherein the krill oil contains at least the compounds:
| | |
|---|---|
| Total phospholipids | >= 56 g/100 g |
| Total omega 3 fatty acids | >= 27 g/100 g |
| Choline | >= 7 g/100 g |

6. A composition according to any of claims 1 to 5 comprising an additional choline salt.

7. A composition according to any of claims 1 to 6 wherein the milk thistle extract contains 65-85% by weight of silymarin.

8. A composition according to any of claims 1 to 7 for use in the treatment or prophylaxis of a liver disease or disorder in a subject.

9. A composition according to any of claims 1 to 7 for use in the treatment or prophylaxis of a bile disease or disorder in a subject.

10. A composition of any of claims 1 to 8 for use in the treatment or prophylaxis of a liver disease or disorder in a subject selected from the group consisting of hepatitis, Steatotic Liver Disease (SLD), Metabolic Dysfunction-associated Steatotic Liver Disease (MASLD), Metabolic Dysfunction-associated Steatohepatitis (MASH), Alcoholic Liver Disease (ALD) or cirrhosis.

11. A composition of claims 1 to 8 for use in the treatment or prophylaxis of Metabolic Dysfunction-associated Steatohepatitis (MASH).

12. The composition for use according to any of claims 9 to 11 wherein the composition is a drug or a dietary supplement or a balanced diet.

13. Pharmaceutical preparations containing a composition for use according to any of claims 9 to 11 together with suitable carrier substances, in particular in the form of drops, capsules, soft capsules or gel capsules or infusions, preferably for oral applications.

14. Pharmaceutical preparations containing a composition for use according to any of claims 9 to 11 together with excipients and additives.
